# FASCICULE DE BREVET EUROPEEN

(11) **EP 0 594 479 B1**
(45) Date de publication et mention de la délivrance du brevet: **04.02.1998**
(21) Numéro de dépôt: 93402521.4
(22) Date de dépôt: 13.10.1993
(51) Int. Cl.: E21B 37/06, C10L 3/00, C07C 7/20

(54) **Procédé pour réduire la tendance à l'agglomération des hydrates dans les effluents de production**
Verfahren zur Reduktion der Agglomerationsneigung von Hydraten in Produktionsausströmungen
Process to reduce the tendency to agglomerate of hydrates in production effluents

(30) Priorité: 23.10.1992 FR 9212836
(43) Date de publication de la demande: 27.04.1994
(73) Titulaire: INSTITUT FRANCAIS DU PETROLE, 92502 Rueil-Malmaison (FR)
(72) Inventeur: Thomas, Michel, F-92500 Rueil Malmaison (FR); Baley, Anne-Sophie, F-75006 Paris (FR); Durand, Jean-Pierre, F-78400 Chatou (FR)

(56) Documents cités:
- EP-A- 0 323 307
- EP-A- 0 323 774
- GB-A- 2 036 594
- GB-A- 2 216 573
- US-A- 2 978 026
- US-A- 2 979 528
- US-A- 3 185 217
- CHEMICAL ABSTRACTS, vol. 106, no. 16, Avril 1987, Columbus, Ohio, US; abstract no. 122892v, KULIEV ET AL. 'PREVENTION OF HYDRATE FORMATION IN NATURAL GAS' page 190 ;colonne 2 ;

## Description

L'invention concerne un procédé pour réduire la tendance à l'agglomération des hydrates de gaz naturel, de gaz de pétrole ou d'autres gaz par utilisation d'au moins un additif. Les gaz qui forment des hydrates peuvent notamment comprendre au moins un hydrocarbure choisi parmi le méthane, l'éthane, l'éthylène, le propane, le propène, le n-butane et l'iso-butane, et éventuellement de l'H₂S et/ou du CO₂.

Ces hydrates se forment lorsque de l'eau se trouve en présence du gaz, soit à l'état libre, soit à l'état dissous dans une phase liquide, telle qu'un hydrocarbure liquide, et lorsque la température atteinte par le mélange notamment d'eau, de gaz et éventuellement d'hydrocarbures liquides, tels que de l'huile, devient inférieure à la température thermodynamique de formation des hydrates, cette température étant donnée pour une composition des gaz connue et lorsque leur pression est fixée.

La formation d'hydrates peut être redoutée, notamment dans l'industrie pétrolière et gazière, pour lesquelles les conditions de formation d'hydrates peuvent être réunies. En effet, pour diminuer le coût de production du pétrole brut et du gaz, tant au point de vue des investissements qu'au point de vue de l'exploitation, une voie envisagée, notamment en production en mer, est de réduire, voire de supprimer, les traitements appliqués au brut ou au gaz à transporter du gisement à la côte et notamment de laisser toute ou une partie de l'eau dans le fluide à transporter. Ces traitements en mer s'effectuent en général sur une plate-forme située en surface à proximité du gisement, de manière que l'effluent, initialement chaud, puisse être traité avant que les conditions thermodynamiques de formation des hydrates ne soient atteintes du fait du refroidissement de l'effluent avec l'eau de mer.

Cependant, comme cela arrive pratiquement, lorsque les conditions thermodynamiques requises pour former des hydrates sont réunies, l'agglomération des hydrates entraîne le remplissage et le blocage des conduites de transport par création de bouchons qui empêchent tout passage de pétrole brut ou de gaz.

La formation de bouchons d'hydrates peut entraîner un arrêt de la production et provoquer ainsi des pertes financières importantes. De plus, la remise en service de l'installation, surtout s'il s'agit de production ou de transport en mer, peut être longue, car la décomposition des hydrates formés est très difficile à réaliser. En effet, lorsque la production d'un gisement sous-marin de gaz naturel ou de pétrole et de gaz comportant de l'eau atteint la surface du sol marin et est ensuite transportée au fond de la mer, il arrive par l'abaissement de la température de l'effluent produit, que les conditions thermodynamiques soient réunies pour que des hydrates se forment, s'agglomèrent et bloquent les conduites de transfert. La température au fond de la mer peut être, par exemple, de 3 ou 4°C.

Des conditions favorables à la formation d'hydrates peuvent aussi être réunies de la même façon à terre, pour des conduites pas ou trop faiblement enfouies dans le sol terrestre, lorsque par exemple la température de l'air ambiant est basse.

Pour éviter ces inconvénients, on a cherché, dans l'art antérieur, à utiliser des produits qui, ajoutés au fluide, pourraient agir comme inhibiteurs en abaissant la température thermodynamique de formation des hydrates. Ce sont notamment des alcools, tels que le méthanol, ou des glycols, tels que le mono-, le di- ou le tri-éthylèneglycol. Cette solution est très onéreuse car la quantité d'inhibiteurs à ajouter peut atteindre 10 à 50 % de la teneur en eau et ces inhibiteurs sont difficiles à récupérer complètement.

On a également préconisé l'isolation des conduits de transport, de manière à éviter que la température du fluide transporté n'atteigne la température de formation des hydrates dans les conditions opératoires. Une telle technique est, elle aussi, très coûteuse.

Par ailleurs, divers composés tensio-actifs non ioniques ou anioniques ont été testés pour leur effet de retardement de la formation d'hydrates au sein d'un fluide renfermant un gaz, notamment un hydrocarbure, et de l'eau. On peut citer par exemple l'article de Kuliev et al : "Surfactants studied as hydrate-formation inhibitors" Gazovoe Delo n° 10 1972, 17-19, rapporté dans Chemical Abstracts 80, 1974, 98122r.

On a encore décrit l'utilisation d'additifs capables de modifier le mécanisme de formation des hydrates, puisque au lieu de s'agglomérer rapidement les uns aux autres et de former des bouchons très solides, les hydrates formés se dispersent dans le fluide sans s'agglomérer et sans obstruer les conduites, tant que la température du fluide transporté n'est pas trop basse.

On peut citer, à cet égard : la demande de brevet EP-A-323774 au nom de la demanderesse, qui décrit l'utilisation de composés amphiphiles nonioniques choisis parmi les esters de polyols et d'acides carboxyliques, substitués ou non-substitués, et les composés à fonction imide; la demande de brevet EP-A-323775, également au non de la demanderesse, qui décrit notamment l'utilisation de composés appartenant à la famille des diéthanolamides d'acides gras ou de dérivés d'acides gras; le brevet US-A-4856593 qui décrit l'utilisation de composés tensio-actifs tels que des phosphonates organiques, des esters phosphates, des acides phosphoniques, leurs sels et leurs esters, des polyphosphates inorganiques et leurs esters, ainsi que des polyacrylamides et des polyacrylates ; et la demande de brevet EP-A-457375, qui décrit l'utilisation de composés tensio-actifs anioniques, tels que les acides alkylarylsulfoniques et leurs sels de métaux alcalins.

Ces composés sont bien adaptés lorsque la phase liquide est formée d'eau et d'hydrocarbures liquides, comme par exemple un condensat ou une huile, mais s'avèrent moins performants lorsque cette phase liquide est constituée majoritairement ou uniquement d'eau (cas des gaz secs).

Il a maintenant été découvert que l'association de polymères et copolymères à base d'oxyde d'alkylène, qui jusqu'à présent n'étaient pas utilisés dans ce but, et d'un inhibiteur de formation des hydrates utilisé à plus faible concentration que celle nécessaire pour inhiber toute formation d'hydrates, présente une excellente efficacité pour modifier le mécanisme de formation des hydrates, tout en limitant la quantité d'hydrates formés et leur tendance à l'agglomération.

Cette modification du mécanisme de formation des cristaux d'hydrates peut notamment être mise à profit pour le transport des fluides formant des hydrates, particulièrement dans les cas où la teneur en eau dans la phase liquide est élevée. Il a en effet été observé que, en présence des additifs de l'invention, les cristaux une fois formés, au lieu de s'agglomérer les uns aux autres et de former des bouchons très solides ou des dépôts dans les appareils où circule le fluide, restent sous forme dispersée et ceci dans une large gamme de température, inférieure à la température thermodynamique d'équilibre à la pression considérée.

Lorsque l'on soumet un mélange de gaz et d'eau à une température notablement plus basse que la température d'équilibre thermodynamique, il se crée un épaississement assez rapide du fluide jusqu'à la formation d'un bouchon solide rendant la circulation du fluide difficile, voire impossible.

L'ajout des additifs de l'invention associés à un inhibiteur thermodynamique à concentration faible ou modérée, particulièrement lorsque la teneur en eau dans la phase liquide est importante, se traduit par une modification de la formation des hydrates, c'est-à-dire que l'on observe la formation de petits cristaux qui restent dispersés plutôt que la formation de bouchons solides.

Ainsi, la présente invention propose un procédé pour réduire la tendance à l'agglomération des hydrates au sein d'un fluide comprenant au moins de l'eau, éventuellement des hydrocarbures liquides, l'eau représentant au moins 30% en volume de l'ensemble eau + hydrocarbures liquides, et des hydrocarbures gazeux susceptibles de former des hydrates, dans des conditions de températures et de pression où ces hydrates peuvent se former, caractérisé en ce qu'on incorpore audit fluide un polymère ou copolymère hydrosoluble à base d'oxyde d'alkylène contenant au moins une séquence -(OR)-ₙ dans laquelle R représente un groupement hydrocarboné de 2 ou 3 atomes de carbone et n représente le degré moyen de polumérisation de ladite séquence, au moins une des séquences -(OR)-ₙ étant une séquence polyoxyde d'éthylène -(CH₂-CH₂-O)ₙ-, en association avec au moins un inhibiteur thermodynamique de formation des hydrates, employé à une concentration inférieure à celle qui serait nécessaire pour inhiber toute formation d'hydrates dans les conditions de pression et de température rencontrées.

L'inhibiteur thermodynamique utilisé peut être en particulier le méthanol, le mono-, di- ou tri-éthylèneglycol. Les sels éventuellement contenus dans l'eau produite peuvent aussi jouer ce rôle. L'inhibiteur permet de limiter la quantité d'hydrates pouvant se former au sein de l'effluent liquide.

Cette association trouve son intérêt lorsque la phase liquide est constituée majoritairement ou totalement d'eau. En effet, en l'absence d'inhibiteur, et après formation des hydrates, la quantité de phase liquide résiduelle sera trop faible pour assurer leur transport.

La quantité d'inhibiteur à introduire pourra être équivalente au plus aux 2/3, de préférence à environ la moitié de la quantité minimale nécessaire pour empêcher toute formation d'hydrates dans les conditions de pression et de température rencontrées et pour le fluide considéré. Cette teneur pourra cependant être optimisée en fonction de la quantité d'additif dispersant employée conjointement.

De préférence, les polymères et copolymères de l'invention sont obtenus à partir d'oxyde d'éthylène et éventuellement d'oxyde de propylène. Il pourra s'agir de polyéthylèneglycols de formule générale :

H -(OCH₂ - CH₂)-ₙ OH

obtenus par réaction de l'oxyde d'éthylène avec de l'éthylèneglycol ou de l'eau. Il pourra également s'agir d'un monoéther, tel que le monométhyléther desdits polyéthylèneglycols. Il pourra également s'agir de copolymères séquencés d' oxyde d'éthylène et d'oxyde de propylène dans lesquels la proportion des motifs oxyde d'éthylène est prépondérante par rapport à celle des motifs oxyde de propylène.

De tels copolymères séquencés peuvent être par exemple obtenus par condensation d'oxyde d'éthylène sur un polypropylèneglycol.

De tels copolymères séquencés peuvent être également obtenus par condensation d'oxyde de propylène sur une molécule d'éthylène diamine suivi de la condensation d'oxyde d'éthylène.

La teneur en oxyde d'éthylène dans ces différents copolymères sera suffisante pour obtenir des copolymères hydrosolubles et sera par exemple supérieure à environ 20 % en masse, de préférence supérieure à environ 50% en masse.

La masse moléculaire des polymères et copolymères utilisés dans l'invention est variable et peut aller de 1.000 jusqu'à 30.000.

Tous ces composés peuvent être utilisés seuls ou en mélange, éventuellement associés à d'autres composés, par exemple des tensio-actifs.

L'emploi selon l'invention des polymères et copolymères tels que décrits précédemment, est particulièrement avantageux du point de vue économique, puisque ces produits sont utilisés à de très faibles concentrations, en général inférieures à 2 % en masse, par exemple de 0,1 à 2 % en masse et de préférence de 0,2 à 1 % en masse, par rapport à l'eau et que le coût de ces produits reste modéré.

Dans le cas où on ajoute au fluide, en tant qu'inhibiteur thermodynamique de formation d'hydrates, un alcool ou un glycol, la teneur en ces derniers pourra aller par exemple jusqu'à 20 % en masse par rapport à l'eau et préférentiellement jusqu'à 10 % en masse.

Dans le cas où le fluide comprend de l'eau de mer ou de l'eau de formation ayant par exemple une teneur en sels supérieure à 20 g/l, l'addition d'alcool ou de glycol n'est pas nécessaire, les sels, par exemple, le chlorure de sodium jouant le rôle d'inhibiteur.

Un mode particulièrement avantageux d'utilisation des polymères et copolymères de l'invention concerne le cas où le mélange d'eau et d'hydrocarbures liquides contient une teneur en eau supérieure à 50 % en volume.

### Exemples.

Dans les exemples qui suivent, pour évaluer l'efficacité des additifs hydrosolubles considérés sur les conditions de formation et sur le maintien en suspension ou le retard à la prise en masse, on a procédé à des essais de formation d'hydrates à partir de méthane et d'eau, en présence éventuellement d'un condensat léger et/ou d'un inhibiteur thermodynamique classique tel que le méthanol.

L'appareillage utilisé pour ces essais comprend une cellule visuelle en saphir, de volume interne 90 cm³, pouvant travailler à une pression maximale de 150 bars. Cette cellule est dans une enceinte thermorégulée, dans laquelle il est possible de maintenir la température constante, ou au contraire de réaliser une descente programmée de celle-ci. La température et la pression dans la cellule sont mesurées par deux capteurs. L'agitation dans cette cellule est réalisée par un agitateur à pales, pouvant tourner à vitesse constante par entraînement magnétique. La valeur du couple de rotation, pour maintenir cette vitesse constante lors de la formation des hydrates, peut être indirectement reliée à la viscosité du milieu.

Une bouteille tampon contenant du méthane est reliée en permanence à la cellule précédente par l'une de ses extrémités, et à une pompe volumétrique par l'autre. Cette pompe permet, par injection de mercure dans la bouteille tampon, de déplacer le volume de méthane nécessaire pour maintenir la pression constante dans cette bouteille et dans la cellule, afin de compenser la chute de pression due à la consommation de gaz lors de la formation des hydrates. La mesure du volume de mercure introduit permet de connaître la quantité de gaz consommé, c'est-à-dire, en négligeant les variations de volume, la quantité d'hydrates formés en fonction du temps ou de la température.

Initialement, la cellule contient 40 cm³ d'eau distillée, ne contenant pas d'additif ou contenant l'additif à tester, et du gaz (méthane), et est en permanence reliée à la bouteille tampon. La pression pour chaque essai est fixée et maintenue constante à 80 bars pendant toute la durée de l'essai. La température initiale est de 20°C.

Pour juger de l'efficacité des additifs, on réalise une descente en température jusqu'à la température de l'expérience, sous agitation constante. L'apparition des cristaux d'hydrates se traduit par une injection de mercure dans la cellule tampon contenant le gaz, ceci afin de maintenir la pression constante, pour compenser la consommation de gaz due à la formation des cristaux d'hydrates. Le volume de gaz injecté compense l'effet de la descente de température et la consommation due à la formation des hydrates. Il peut être relié à la quantité d'hydrates formés. La valeur du couple de rotation est également enregistrée pendant toute la durée de l'essai. Lorsque les cristaux d'hydrates s'agglomèrent, l'agitateur se bloque complètement.

La valeur initiale du couple de rotation est de 17,5 mN.m pour une vitesse de rotation de l'agitateur de 300 tours/minute.

La température de dissociation des hydrates de méthane à 80 bars est de 10,7°C en présence d'eau pure, et de 5,9°C en présence d'eau avec 10 % poids de méthanol.

Pour empêcher toute formation d'hydrates à 80 bars et 2°C (conditions des expériences), il faudrait ajouter à l'eau environ 17 % poids de méthanol.

Entre deux essais consécutifs la cellule est nettoyée avec de l'eau distillée puis du méthanol, et est ensuite séchée. La durée maximale d'un essai est fixée à 8 heures.

Les exemples suivants illustrent l'invention sans toutefois en limiter la portée. Les exemples 1, 2, 3, 5, 6 et 7 sont donnés à titre de comparaison.

### Exemple 1 (comparatif).

Dans cet exemple, on opère avec 40 cm³ d'eau distillée et du méthane, sans aucun additif. La pression est fixée à 80 bars et la température à 2°C, suivant le protocole expérimental décrit ci-dessus.

Dans ces conditions, on assiste à un blocage de l'agitateur 15 minutes après le début de la formation des hydrates. Le volume de gaz consommé est de 38.6 cm³.

### Exemple 2 (comparatif).

Dans cet exemple, on opère comme dans l'exemple 1, avec le même gaz et la même eau, sous la même pression, mais on ajoute à l'eau 0.5 % poids du copolymère oxyde d'éthylène-oxyde de propylène obtenu par condensation d'oxyde de propylène sur une molécule d'éthylène diamine suivie de la condensation d'oxyde d'éthylène, ce copolymère ayant une masse moléculaire moyenne de 26.000 et contenant 80 % en masse d'oxyde d'éthylène.

Dans ces conditions, on assiste à un blocage de l'agitateur 1,5 heure après le début de la formation des hydrates. Le volume de gaz consommé est de 35.1 cm³

### Exemple 3 (comparatif).

On opère comme dans l'exemple 1, mais on ajoute à l'eau 10 % poids de méthanol.

Dans ces conditions, on assiste au blocage de l'agitateur 4 heures après le début de la formation des hydrates. Le volume de gaz consommé est de 36,4 cm³.

### Exemple 4.

On opère comme dans l'exemple 1, mais on ajoute à l'eau 10 % poids de méthanol et 0,5 % poids du copolymère utilisé dans l'exemple 2.

Dans ces conditions, on observe une légère augmentation de la valeur du couple de rotation qui se stabilise à 18,6 mN.m pendant 8 heures. Le volume de gaz consommé est de 36,5 cm³.

### Exemple 5 (comparatif).

On opère comme dans l'exemple 1, mais on part de 30 cm³ d'eau et de 10 cm³ d'un condensat léger de Mer du Nord, sans aucun additif.
Dans ces conditions, on assiste à un dépôt progressif des hydrates sur les parois de la cellule puis à un blocage de l'agitateur 0,75 heure après le début de la formation des hydrates. Le volume de gaz consommé est de 33,7 cm³.

### Exemple 6 (comparatif).

On opère comme dans l'exemple 5, mais on part de 30 cm³ d'eau et de 10 cm³ d'un condensat léger de Mer du Nord, et on ajoute 0,5 % poids par rapport à l'eau de monométhyléther de polyéthylèneglycol, ce produit ayant une masse moléculaire moyenne voisine de 5.000.

Dans ces conditions, on assiste à une augmentation de la valeur du couple qui se stabilise à 29,5 mN.m après 5 heures, puis finalement à un blocage après 7 heures. Le volume de gaz ajouté est de 32,6 cm³.

### Exemple 7 (comparatif).

On opère comme dans l'exemple 5, mais on ajoute à l'eau 10 % poids de méthanol.

Dans ces conditions, on assite à un blocage de l'agitateur 3,5 heures après le début de la formation des hydrates. Le volume de gaz ajouté est de 33,9 cm³.

### Exemple 8.

On opère comme dans l'exemple 6, mais on ajoute à l'eau 10 % poids de méthanol.

Dans ces conditions, on assiste à une augmentation de la valeur du couple qui se stabilise à 19,5 mN.m après 6 heures, sans blocage de l'agitation. Le volume de gaz injecté est de 34,6 cm³.

### Exemple 9.

Si dans l'exemple 8 on ajoute seulement 0,2 % poids par rapport à l'eau de monométhyléther de polyéthylèneglycol, on asiste à une augmentation de la valeur du couple qui se stabilise à 25,3 mN.m après 4 heures, sans blocage de l'agitation. Le volume de gaz injecté est de 32,8 cm³.

### Exemple 10.

On opère comme dans l'exemple 2, mais on utilise de l'eau salée (NaCl à 50 g/l).

Dans ces conditions, on assiste à une augmentation de la valeur du couple qui se stabilise à 24,5 mN.m après 4 heures, sans blocage de l'agitation. Le volume de gaz consommé est de 31,9 cm³.

## Revendications

1. Procédé pour réduire la tendance à l'agglomération des hydrates au sein d'un fluide comprenant au moins de l'eau, éventuellement des hydrocarbures liquides, l'eau représentant au moins 30% en volume de l'ensemble eau + hydrocarbures liquides, et des hydrocarbures gazeux susceptibles de former des hydrates, dans des conditions de températures et de pression où ces hydrates peuvent se former, caractérisé en ce qu'on incorpore audit fluide un polymère ou copolymère hydrosoluble à base d'oxyde d'alkylène contenant au moins une séquence -(OR)-ₙ dans laquelle R représente un groupement hydrocarboné de 2 ou 3 atomes de carbone et n représente le degré moyen de polymérisation de ladite séquence, au moins une des séquences -(OR)-ₙ étant une séquence polyoxyde d'éthylène - (CH₂-CH₂-O)ₙ-, en association avec au moins un inhibiteur thermodynamique de formation des hydrates, employé à une concentration inférieure à celle qui serait nécessaire pour inhiber toute formation d'hydrates dans les conditions de pression et de température rencontrées.

2. Procédé selon la revendication 1, caractérisé en ce que ledit polymère hydrosoluble est un polyéthylèneglycol.

3. Procédé selon la revendication 1, caractérisé en ce que ledit polymère hydrosoluble est un monoéther de polyéthylèneglycol.

4. Procédé selon la revendication 1, caractérisé en ce que ledit copolymère hydrosoluble est un copolymère séquencé d'oxyde d'éthylène et d'oxyde de propylène dans lequel la proportion des motifs oxyde d'éthylène est prépondérante par rapport à celle des motifs oxyde de propylène.

5. Procédé selon la revendication 4, caractérisé en ce que ledit copolymère hydrosoluble est obtenu par condensaticn d'oxyde d'éthylène sur un polypropylèneglycol.

6. Procédé selon la revendication 4, caractérisé en ce que ledit copolymère hydrosoluble est obtenu par condensation d'oxyde de propylène sur une molécule d'éthylène diamine suivi de la condensation d'oxyde d'éthylène.

7. Procédé selon l'une des revendications 1 à 6, caractérisé en ce que la teneur dudit polymère ou copolymère en oxyde d'éthylène est supérieure à environ 20 % en masse.

8. Procédé selon l'une des revendications 1 à 7, caractérisé en ce que la masse moléculaire dudit polymère ou copolymère hydrosoluble est comprise entre 1 000 et 30 000.

9. Procédé selon l'une des revendications 1 à 8, caractérisé en ce que ledit polymère ou copolymère hydrosoluble est utilisé à une concentration de 0,1 à 2 % en masse par rapport à l'eau présente.

10. Procédé selon l'une des revendications 1 à 9, caractérisé en ce que l'inhibiteur thermodynamique de formation des hydrates est le méthanol, le mono-, le di- ou le triéthylèneglycol.

11. Procédé selon l'une des revendications 1 à 10, caractérisé en ce que la concentration en inhibiteur dans la phase aqueuse est inférieure aux deux tiers de la concentration nécessaire pour inhiber totalement la formation des hydrates.

12. Procédé selon l'une des revendications 1 à 11, caractérisé en ce que la teneur en inhibiteur est d'au plus 20% en masse par rapport à l'eau.

13. Procédé selon l'une des revendications 1 à 12, caractérisé en ce que, l'eau présente dans le fluide est une eau de gisement contenant au moins un sel tel que le chlorure de sodium, et on se dispense au moins partiellement de l'addition d'un inhibiteur thermodynamique.

## Patentansprüche

1. Verfahren zum Verringern der Verklumpungsneigung von Hydraten in einem Fluid, das wenigstens Wasser, gegebenenfalls flüssige Kohlenwasserstoffe, wobei das Wasser mindestens 30 Vol.-% des Systems Wasser + flüssige Kohlenwasserstoffe darstellt, und gasförmige Kohlenwasserstoffe, die Hydrate bilden können, umfaßt, unter Temperatur- und Druckbedingungen, unter denen diese Hydrate sich bilden können, dadurch gekennzeichnet, daß dem Fluid ein wasserlösliches Polymer oder Copolymer auf Grundlage von Alkylenoxid, das wenigstens eine Sequenz -(OR)-ₙ enthält, in der R eine Kohlenwasserstoffgruppe von 2 oder 3 Kohlenstoffatomen und n den mittleren Polymerisierungsgrad der Sequenz bezeichnen, wobei wenigstens eine der Sequenzen -(OR)-ₙ eine Polyethylenoxidsequenz -(CH₂-CH₂-O)ₙ- ist, in Verbindung mit wenigstens einem thermodynamischen Hydratbildungsinhibitor beigegeben wird, der bei einer niedrigeren Konzentration verwendet wird, als notwendig wäre, um jegliche Hydratbildung unter den vorgefundenen Druck- und Temperaturbedingungen zu verhindern.

2. Verfahren nach Anspruch 1, dadurch gekennzeichnet, daß das wasserlösliche Polymer ein Polyethylenglycol ist.

3. Verfahren nach Anspruch 1, dadurch gekennzeichnet, daß das wasserlösliche Polymer ein Polyethylenglycol-Monoether ist.

4. Verfahren nach Anspruch 1, dadurch gekennzeichnet, daß das wasserlösliche Polymer ein sequenziertes Ethylenoxid-Propylenoxid-Copolymer ist, bei dem der Anteil der Ethylenoxidmuster im Vergleich zu dem der Propylenoxidmuster überwiegt.

5. Verfahren nach Anspruch 4, dadurch gekennzeichnet, daß das wasserlösliche Polymer durch Kondensation von Ethylenoxid an einem Polypropylenglycol erhalten wird.

6. Verfahren nach Anspruch 4, dadurch gekennzeichnet, daß das wasserlösliche Copolymer durch Kondensation von Propylenoxid an einem Ethylendiaminmolekül, gefolgt von der Kondensation von Ethylenoxid, erhalten wird.

7. Verfahren nach einem der Ansprüche 1 bis 6, dadurch gekennzeichnet, daß der Gehalt des Polymers oder Copolymers an Ethylenoxid größer als ca. 20 Gew.-% ist.

8. Verfahren nach einem der Ansprüche 1 bis 7, dadurch gekennzeichnet, daß die Molekülmasse des wasserlöslichen Polymers oder Copolymers zwischen 1000 und 30000 einschließlich liegt.

9. Verfahren nach einem der Ansprüche 1 bis 8, dadurch gekennzeichnet, daß das wasserlösliche Polymer oder Copolymer mit einer Konzentration von 0,1 bis 2 Gew.-% im Verhältnis zum vorhandenen Wasser verwendet wird.

10. Verfahren nach einem der Ansprüche 1 bis 9, dadurch gekennzeichnet, daß der thermodynamische Hydratbildungsinhibitor Methanol, Mono-, Di- oder Triethylenglycol ist.

11. Verfahren nach einem der Ansprüche 1 bis 10, dadurch gekennzeichnet, daß die Konzentration an Inhibitor in der wäßrigen Phase kleiner als zwei Drittel derjenigen Konzentration ist, die notwendig ist, um die Bildung von Hydraten vollständig zu inhibieren.

12. Verfahren nach einem der Ansprüche 1 bis 11, dadurch gekennzeichnet, daß der Gehalt an Inhibitor höchstens 20 Gew.-% im Verhältnis zum Wasser ist.

13. Verfahren nach einem der Ansprüche 1 bis 12, dadurch gekennzeichnet, daß das im Fluid vorhandene Wasser ein Lagerstättenwasser ist, das wenigstens ein Salz wie etwa Natriumchlorid enthält, und daß von der Zufügung eines thermodynamischen Inhibitors wenigstens teilweise abgesehen wird.

## Claims

1. Process for reducing the agglomeration tendency of hydrates within a fluid incorporating at least water, optionally liquid hydrocarbons, water representing at least 30 % by volume of the mixture water + liquid hydrocarbons, and gaseous hydrocarbons able to form hydrates, under temperature and pressure conditions where said hydrates can form, characterized in that into said fluid is incorporated an alkylene oxide-based hydrosoluble polymer or copolymer containing at least one -(OR)-ₙ sequence, in which R represents a hydrocarbon group having or 3 carbon atoms and n represents the average degree of polymerization of said sequence, at least one of the -(OR)-ₙ sequences being an polyethylene oxide sequence, -(CH2-CH₂-O)-ₙ , combined with at least one thermodynamic hydrate formation inhibitor, used in a concentration below that which would be necessary for inhibiting any hydrate formation under the pressure and temperature conditions encountered.

2. Process according to claim 1, characterized in that said hydrosoluble polymer is a polyethylene glycol.

3. Process according to claim 1, characterized in that said hydrosoluble polymer is a polyethylene glycol monoether.

4. Process according to claim 1, characterized in that said hydrosoluble copolymer is a sequenced copolymer of ethylene oxide an propylene oxide in which the proportion of ethylene oxide units preponderates compared with that of the propylene oxide units.

5. Process according to claim 4, characterized in that said hydrosoluble copolymer is obtained by the condensation of ethylene oxide on a polypropylene glycol.

6. Process according to claim 4, characterized in that said hydrosoluble copolymer is obtained by the condensation of propylene oxide on an ethylene diamine molecule followed by ethylene oxide condensation.

7. Process according to any one of the claims 1 to 6, characterized in that the content of said ethylene oxide polymer or copolymer exceeds approximately 20% by weight.

8. Process according to any one of the claims 1 to 7, characterized in that the molecular weight of said hydrosoluble polymer or copolymer is between 1000 and 30,000.

9. Process according to any one of the claims 1 to 8, characterized in that said hydrosoluble polymer or copolymer is used in a concentration of 0.1 to 2% by weight, based on the water present.

10. Process according to any one of the claims 1 to 9, characterized in that the thermodynamic hydrate formation inhibitor is methanol, mono-, di- or tri-ethylene glycol.

11. Process according to any one of the claims 1 to 10, characterized in that the inhibitor concentration in the aqueous phase is below 2/3 of the concentration necessary for completely inhibitin hydrate formation.

12. Process according to any one of the claims 1 to 11, characterized in that the inhibitor concentration is at the most 20% by weight, based on the water.

13. Process according to any one of the claims 1 to 12, characterized in that the water present in the fluid is a deposit water containing at least one salt such as sodium chloride, so that at least in part the addition of a thermodynamic inhibitor is dispensed with.
